# EUROPEAN PATENT SPECIFICATION

(11) **EP 1 530 980 B1**
(45) Date of publication and mention of the grant of the patent: **03.01.2018**
(21) Application number: 04256741.2
(22) Date of filing: 01.11.2004
(51) Int. Cl.: A61M 16/18

(54) **Anaesthetic vaporiser**
Narkosemittelverdunster
Evaporateur d'anesthésique

(30) Priority: 11.11.2003 GB 0326298
(43) Date of publication of application: 18.05.2005
(73) Proprietor: Fiedorowicz, Richard John, Abingdon, Oxfordshire OX14 3RY (GB)
(72) Inventor: Fiedorowicz, Richard John, Abingdon, Oxfordshire OX14 3RY (GB)
(74) Representative: Franks & Co (South) Limited

(56) References cited:
- GB-A- 1 088 080
- GB-A- 1 230 972
- US-A- 3 575 168
- US-A- 4 059 657
- US-A- 4 075 297
- US-A- 4 444 182
- US-A- 4 879 997

## Description

The present invention relates to a device for vaporising anaesthetic liquids for administration in a gas stream to a patient. More particularly, but not exclusively, it relates to an anaesthetic vaporiser of the by-pass type with improved consistency of delivery of anaesthetic vapour concentrations and improved anaesthetic reservoir capacity.

Many anaesthetic agents used in general anaesthesia are liquids under standard conditions, and are administered as vapours, borne on a flow of carrier gas, such as air, pure oxygen or mixtures of oxygen and gaseous anaesthetics, such as nitrous oxide. Commonly used anaesthetics administered in this fashion include halothane (CHBrCℓ-CF₃), enflurane (CHF₂-O-CF₂-CHFCℓ), isoflurane (CHF₂-O-CHCℓ-CF₃) and sevoflurane (CH₂F-O-CH(CF₃)₂).

An anaesthetic vaporiser contains a reservoir of liquid anaesthetic, over or through which is passed a stream of carrier gas, which picks up anaesthetic vapour. The carrier gas stream is delivered to the vaporiser by an anaesthetic machine, and is a blend of oxygen, nitrous oxide, and/or air to produce a mixture with exactly the required composition for administration to the patient through a patient breathing circuit.

It is clearly highly desirable for an anaesthetist to be able to rely on a consistent supply of anaesthetic vapour at a known and predeterminable concentration, so that he may concentrate on monitoring the patient and where necessary changing the gas blend at the anaesthetic machine in response to changes in the patient's condition.

A type of vaporiser which has been found to be particularly suitable is the by-pass vaporiser. In this, the carrier gas stream is split into two. A first stream is passed across the liquid anaesthetic in its reservoir to become saturated with anaesthetic vapour, and is then recombined with the second stream which has bypassed the reservoir. In theory, this should produce an output carrier gas stream with a known concentration of vapour.

However, the saturated vapour pressure of a liquid depends on the temperature. Not only may ambient temperatures vary, but the latent heat of vaporisation of the liquid leads to it cooling significantly as vapour is formed and borne off in the carrier gas. It is hence customary to control the recombination of the bypass gas stream and the gas stream saturated with vapour by using a temperature sensitive valve mechanism, for example controlled by a bimetallic strip. Thus, when the saturated gas stream has become colder, and so contains a lower saturated vapour concentration, the valve relatively restricts the flow of the bypass gas stream, so that the resulting recombined gas stream remains at the desired vapour concentration. (A separate needle valve arrangement is normally provided, by which an anaesthetist may select a desired vapour concentration).

It is hence very important that the temperature sensing means of the valve is responsive to the exact anaesthetic temperature. Vaporisers have been developed in which the valve and temperature sensing means are positioned immediately below a base of the anaesthetic reservoir. A drawback of such an arrangement is the temperature difference between the anaesthetic liquid and gas, which can amount to as much as twenty degrees Celsius, although this depends, for example, on the carrier gas flow rate.

An additional problem with this arrangement is that access to the bypass valve for adjustment is very awkward, possibly requiring draining of the reservoir or even dismantling the vaporiser completely.

It is hence an object of the present disclosure to provide a control arrangement for a by-pass anaesthetic vaporiser which is more accurately responsive to vapour temperatures and hence actual saturated vapour concentrations, and which is easier to adjust.

Another source of potential variation in supplied vapour concentration is that the carrier gas stream passing through the reservoir may not have a sufficient residence time to become fully saturated with anaesthetic vapour. Various wick arrangements have been proposed with high surface areas to promote rapid transfer of liquid anaesthetic to the vapour phase, as well as indirect gas flow paths to increase residence times and to prevent carrier gas "short cuts" from inlet to outlet of the reservoir. Some proposals have the carrier gas constrained to follow a spiral path, lined with a wick material, from outside to inside, increasing its time in contact with the anaesthetic-soaked wick material. These arrangements and others along similar lines may improve gas/vapour contact, but some problems remain.

For example, the anaesthetic vapour is significantly heavier than air. A concentration gradient may thus be set up between a point immediately above the liquid anaesthetic and a point close to a top of the reservoir. A conventional vaporiser with a reservoir capacity of perhaps 120-250 cm³ will empty in around two to five hours normal use, and thus may require refilling in the course of a prolonged operation. Clearly, the vapour concentration in an upper part of the reservoir can vary greatly and this effect will increase as the liquid level falls in the reservoir. The existing arrangements cannot allow for this, and there is hence a likelihood of a drift or variation in the vapour concentrations produced, particularly at low or high carrier gas flow rates.

It is hence a further object of the present disclosure to provide an apparatus having a carrier gas path and wick arrangement capable of providing improved consistency of vapour saturation, relative to current arrangements.

Although an extended carrier gas path length would clearly be beneficial in this respect, there is a potential drawback. An elaborate structure, designed to define a long path length, may result in poor conduction of heat through the structure. Thus, significant temperature differentials and hence differences in saturated vapour pressure may build up across the structure and the path it defines. It would hence be preferable if the carrier gas path could be defined so as to obviate this risk as much as possible.

Ideally, arrangements should be made to provide warmth to all parts of the path-defining structure, to compensate for the lost latent heat of vaporisation. Thus, the "running temperature" of the vaporiser can be kept relatively high, leading to a relatively high and consistent anaesthetic vapour pressure.

A further problem with existing vaporisers results from the use of a mechanical ventilator to deliver the final gas/anaesthetic mixture to the patient. The pumping action of the ventilator can cause a major pulsation in back-pressure, upstream therefrom. This can have an effect as far upstream as the vaporiser, leading to variations in gas flow. Major carrier gas pressure variations can also lead to significant variations in the saturated vapour pressure of the anaesthetic. Conventionally, a large gas volume can be provided above the liquid anaesthetic in the vaporiser, as a "buffer" against back-pressure pulses, but this is not always sufficient, and is in any case a waste of valuable space within the vaporiser.

It is hence another object of the present disclosure to provide an improved means of obviating the effect of back-pressure variations on the operation of the vaporiser.

As mentioned, space can be critical within a vaporiser. This is because they are constrained in their external dimensions to fit standardised mountings. Conventionally, a set of one, two or three vaporisers, each possibly containing a different anaesthetic, is mounted side by side in the anaesthetic machine.

Thus, a trade-off is usually necessary between the spaces occupied by the various internal components of the vaporiser, which results in a limited reservoir capacity, as mentioned above. The need for the reservoir to be filled periodically during long operations has led to problems. If the anaesthetist is busy monitoring the patient, non-expert personnel may have to fill the reservoir. This has occasionally led to overfilling, and even carry-over of liquid anaesthetic downstream of the vaporiser. There are clear benefits from maximising the reservoir capacity to reduce the need for refilling.

UK Patent No. 1088080 discloses an early example of a bypass anaesthetic vaporiser, in which a first carrier gas path is defined by a set of nested thin-walled cylinders, the walls of which are partially covered with wick material extending into a reservoir of liquid anaesthetic. This vaporiser regulates the proportions of carrier gas passing through the first carrier gas path and the second, bypass path by means of a temperature-sensitive valve controlled by a capsule of highly thermally expansive fluid, submerged in the reservoir of liquid anaesthetic.

UK Patent No. 1230972 discloses another early bypass anaesthetic vaporiser, in which the first carrier gas path is also defined by nested thin-walled cylinders with wick material covering part of their surfaces and extending into a reservoir of liquid anaesthetic. In this vaporiser, the proportion of carrier gas passing through each path is controlled manually.

US Patent No. 4075297 discloses a bypass anaesthetic vaporiser in which a coil of tubing is arranged within an annular evaporation chamber. An external surface of the tubing is covered with wick material, as is part of the walls of the chamber, the wick material extending into a reservoir of liquid anaesthetic. There is a manual control to adjust the proportions of carrier gas following each gas path.

US Patent No. 3575168 discloses a further early bypass anaesthetic vaporiser, in which the carrier gas is passed down a narrow annular passage, across a surface of the liquid anaesthetic and back up a further narrow annular passage lined with wick material. The proportions of gas passing along the above gas path and the bypass path are controlled by a valve operated by a bimetallic strip over which the gas in the bypass path flows.

US Patent No. 4444182 discloses a bypass anaesthetic vaporiser in which a first carrier gas path is formed by a helical downward path, flow across a surface of liquid anaesthetic in a reservoir, and then an upward path defined by a helical coil extending within an annular chamber lined with wick material.

According to the present invention, there is provided an anaesthetic vaporiser comprising reservoir means adapted to hold a supply of liquid anaesthetic agent, a plurality of spaced, generally parallelly-extending channel means, each extending generally vertically in use, to define a path for carrier gas being passed through the vaporiser, and each being provided with respective wick means extending into said reservoir means or operatively connected thereto so as to permit flow of liquid anaesthetic agent from the reservoir means to each wick means.

The anaesthetic agent may thus evaporate from each wick means and enter the carrier gas. Preferably, the channel means are so connected that the carrier gas is directed upwardly along some of said channel means and downwardly along a remainder thereof.

Advantageously, the carrier gas is directed alternately upwardly and downwardly along successive channel means.

The channel means may be connected serially to form an unbranched path for the carrier gas.

Preferably, each wick means is substantially co-extensive with a respective channel means.

Advantageously, each wick means comprises a layer of wick material lining at least part of an inner surface of a respective channel means, optionally a majority thereof.

Each channel means may be provided with frame means adapted to hold said layer of wick material to said surface.

Each channel means may have an elongate generally cylindrical shape.

The frame means may then comprise helical spring means dimensioned to be insertable into a respective channel means with the wick material held between it and the inner surface of the channel means.

The wick means may be operatively connected with the reservoir means by a sheet of second wick material extending into said reservoir means and contacting each wick means.

The second wick material may line a part of the inner surface of each channel means not lined by the respective wick means.

Preferably, the wick means and wick material comprise a non-woven fabric material, such as a felted material.

Advantageously, the wick means and wick material comprise a fluorinated plastics material, such as polytetrafluoroethylene.

The central block may be provided with upper and lower end caps, each provided with passage means to connect a respective end of each channel means to a corresponding end of another channel means, or to inlet or outlet means for the carrier gas.

According to an aspect of the present disclosure, there is provided an anaesthetic vaporiser of the by-pass type, as defined hereinabove, comprising reservoir means adapted to hold a supply of liquid anaesthetic agent in a lower, in use, portion thereof, a first carrier gas path extending through an upper portion of the reservoir means and arranged so that carrier gas passing therethrough may become saturated with anaesthetic vapour, a second carrier gas path avoiding contact of carrier gas with anaesthetic vapour, and temperature sensitive valve means adapted to regulate the proportion of carrier gas flowing along each said path, wherein the valve means is so mounted as to sense and respond to the temperature of carrier gas and anaesthetic vapour adjacent the upper portion of the reservoir.

Preferably, the first carrier gas path is arranged to pass through said upper portion adjacent the valve means.

Advantageously, the valve means comprises a part of the second carrier gas path.

The valve means may comprise a variable restriction in the second carrier gas path, controlled by bimetallic strip means.

Said restriction may comprise a pair of plate means, between which the carrier gas is constrained to flow, a separation between the plate means being controlled by movement of said bimetallic strip means.

According to another aspect of the present disclosure, there is provided an anaesthetic vaporiser of the by-pass type as defined hereinabove, comprising a first carrier gas path arranged so that evaporating anaesthetic agent may saturate carrier gas passing therethrough and a third carrier gas path arranged so that carrier gas flows therethrough before flowing through the first carrier gas path, the first and third carrier gas paths extending each adjacent the other such that heat may flow from carrier gas in the third path to replace that lost by evaporation of anaesthetic into carrier gas in the first path.

Preferably, each of the first and third carrier gas paths extends through a block of thermally conductive material, for example comprising a metal such as aluminium.

Each of the first and third carrier gas paths advantageously comprises a plurality of channel means extending each generally parallelly to the others through the block, optionally connected in series to form a single unbranched path.

According to another aspect of the present disclosure, there is provided an anaesthetic vaporiser provided with buffer means to obviate the effects of variations in back-pressure produced by a mechanical ventilator or the like operatively connected thereto, the buffer means comprising an elongate indirect carrier gas path within the vaporiser.

Preferably, said elongate path comprises a plurality of channel means each extending generally parallelly to the others and so connected, optionally in series, that a direction of flow of gas passing therethrough is substantially reversed between each channel means and its neighbours.

An embodiment of the present invention will now be more particularly described by way of example and with reference to the accompanying drawings, in which:
**Figure 1** is a perspective view, partially sectioned, of a wick assembly of an anaesthetic vaporiser embodying the invention;
**Figure 2** is a perspective view of components of the wick assembly shown in Figure 1, in separated condition;
**Figure 3** is a perspective view of the wick assembly shown in Figure 1 in position in a main body of the vaporiser, partially sectioned;
**Figure 4** is a perspective view from below of an upper manifold block of the vaporiser;
**Figure 5** is a scrap plan view of part of the wick assembly shown in Figure 1, showing details of the wicks thereof;
**Figure 6** is a perspective view of the assembly shown in Figure 1, partially sectioned and with the wicks themselves omitted, to show a flow path therethrough;
**Figure 7** is a perspective view from behind of the main body, front block and upper manifold block of the vaporiser, separated one from each other to show a flow path through a by-pass valve thereof;
**Figure 8** is a frontal elevation of the assembled vaporiser;
**Figure 9** is a plan view from above of the assembled vaporiser;
**Figure 10** is a cross-sectional view of the vaporiser of Figures 8 and 9, taken along the line X-X of Figure 8;
**Figure 11** is a cross-sectional view of the vaporiser of Figures 8 and 9, taken along the line XI-XI of Figure 9; and
**Figure 12** is a cross-sectional view of the vaporiser of Figures 8 and 9, taken along the line XII-XII of Figure 8.

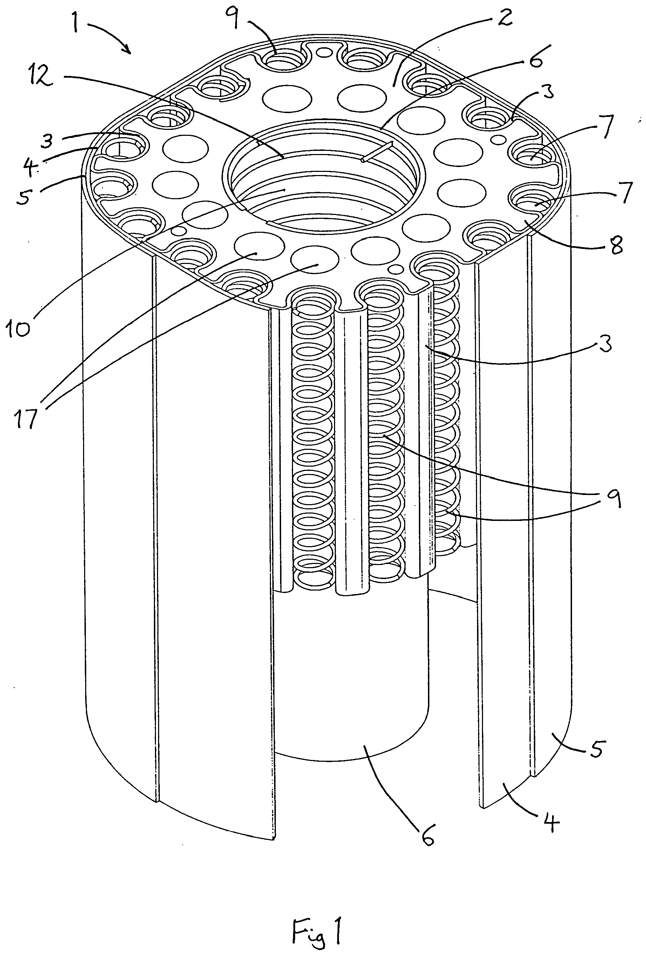

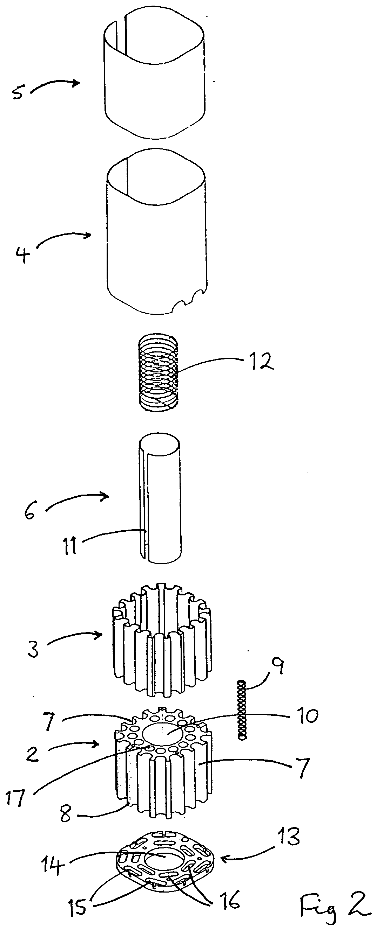

Referring now to the Figures, and to Figures 1 and 2 in particular, a wick assembly 1 of an anaesthetic vaporiser comprises a wick core block 2 of aluminium alloy, an inner wick 3, a main wick 4, an outer wick 5 and a centre wick 6, each wick 3-6 comprising a needle felt wick material made from polytetrafluoroethylene fibres.

The wick core block 2 is generally cube-shaped, with its edges extending vertically, in use, being substantially rounded. A plurality of channels 7 (in this embodiment, seventeen) extend vertically through the vertical faces of the core block 2, separated by a plurality of ribs 8. Each channel 7 has a generally Ω - shaped profile.

The inner wick 3 comprises a sheet of wick material extending around the vertical faces of the core block 2 so as to follow the profile of each channel 7 and rib 8. A helical spring 9 extends along an interior of each channel 7, acting as a frame to hold a respective portion of the inner wick 3 in contact with an interior surface of the channel 7. The inner wick 3 thus lines each channel 7 and is contactable by gas passing therethrough.

The main wick 4 extends around the vertical faces of the core block 2 exterior of the channels 7, contacting the inner wick 3 where it passes over the ribs 8. The main wick 4 also extends downwardly below the wick core block 2, so that it may pick up liquid anaesthetic agent from a reservoir (see below).

The outer wick 5 extends around an outside of the main wick 4. It may either also extend downwardly below the core block 2, as shown in Figure 1, or have a vertical extent generally similar to that of the core block 2 and the inner wick 3, as shown in Figure 2.

The core block 2 is also provided with a substantially cylindrical central aperture 10, extending vertically therethrough. The centre wick 6 extends around a majority of an inner surface of the central aperture 10, having the form of a hollow elongate cylinder with a longitudinal slot 11, and also extends downwardly below the core block 2 so that it may pick up liquid anaesthetic agent from the reservoir.

The centre wick 6 is held in contact with the inner surface of the central aperture 10 by a helical central spring 12 of corresponding dimensions.

The wick assembly 1 also comprises a base plate 13 (omitted from Figure 1 for clarity). This is fastened to an underside of the core block 2, fitting within the main wick 4. The base plate has a central aperture 14, corresponding to the central aperture 10 of the core block 2, through which the centre wick 6 passes. The base plate 13 is provided on its upper face with an outer set of elongate recesses 15 and an inner set of elongate recesses 16, whose function is described below.

The core block 2 is also provided with a plurality of cylindrical passages 17 (in this embodiment, twelve) extending vertically therethrough, located between the channels 7 and the central aperture 10.

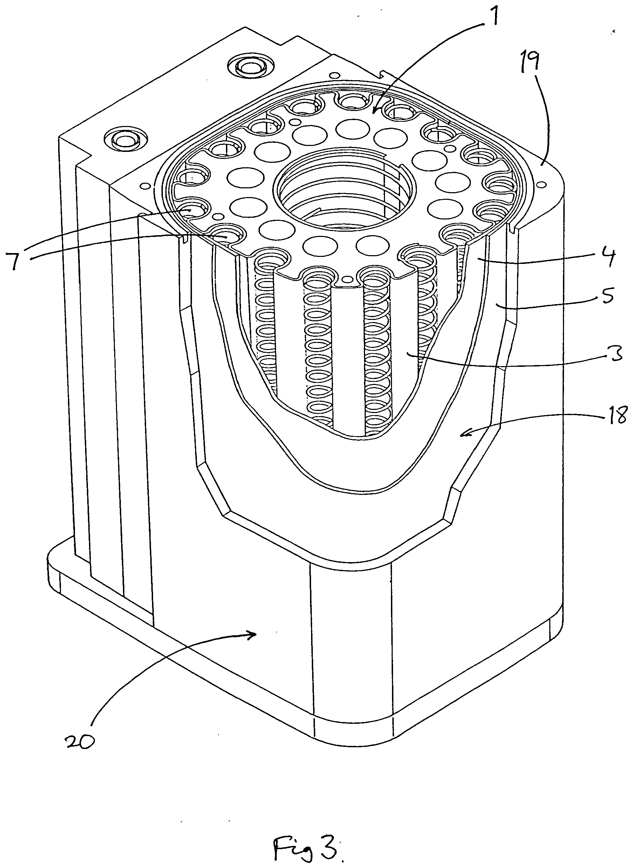

As shown in Figure 3, the wick assembly 1 fits closely inside a reservoir chamber 18, located within a main body 19 of the vaporiser. The main wick 4 and outer wick 5 are held securely between the core block 2 and corresponding walls of the reservoir chamber 18. Thus, each channel 7 adopts a generally cylindrical form, entirely lined with wick material. A lower zone 20 of the reservoir chamber 18 is filled, in use, with a supply of a liquid anaesthetic agent. The main wick 4 and centre wick 6 (and optionally the outer wick 5) extend into the lower zone 20. Thus, by capillary action, liquid anaesthetic agent passes upwardly throughout the main wick 4 and centre wick 6. From the main wick 4, it passes into the portions of the inner wick 3 over the ribs 8 of the core block 2 and thence to the portions of the inner wick 3 lining the channels 7. The anaesthetic is then free to evaporate from the wicks 3, 4, 6 into the respective channels 7 and central aperture 10.

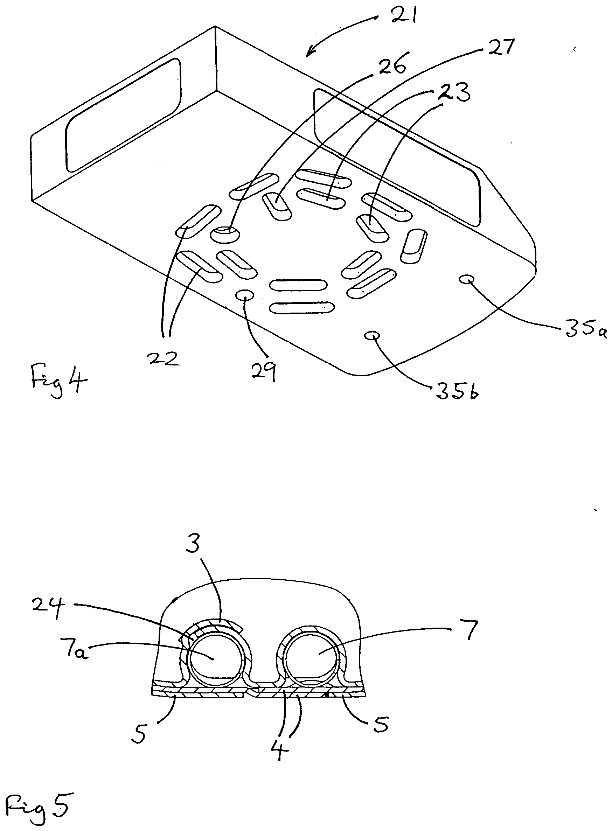

An upper manifold block 21, as shown in Figure 4, is mounted to an upper surface of the wick assembly 1 and main body 19. As for the base plate 13 of the wick assembly 1, the upper manifold block 21 is provided on its surface facing the wick core block 2 with an outer set of elongate recesses 22 and an inner set of elongate recesses 23.

As shown in Figure 5, one channel 7a is provided with a groove 24 extending longitudinally thereof, to receive overlapping extremities of the inner wick 3. The main wick 4 also overlaps with itself at its extremities, as shown, and so the outer wick 5 is sized to leave a gap between its extremities, which receives the overlapping extremities of the main wick 4.

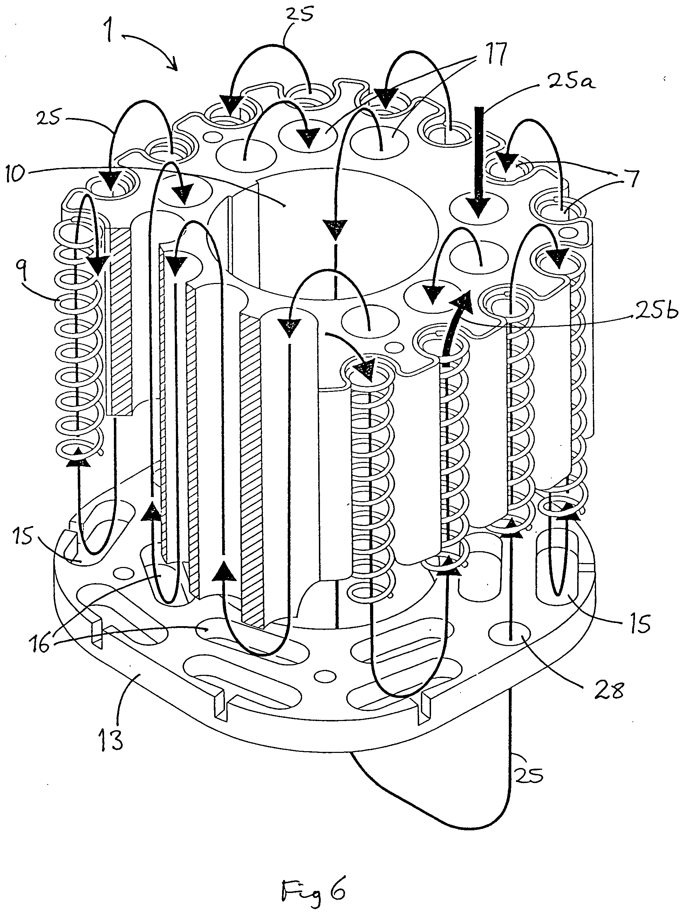

The wick assembly 1 defines a prolonged path in contact with anaesthetic-soaked wick material, along which carrier gas is passed to become fully saturated with anaesthetic vapour. Figure 6 shows this gas path, which follows the sequence of arrows 25.

Carrier gas enters the wick assembly 1 from an entry port 26 of the upper manifold block 21 (see Figure 4), flowing into a passage 17 aligned with the entry port 26, along arrow 25a. At a lower end, this passage 17 is aligned with one of the inner set of elongate recesses 16 of the base plate 13, which connects it to a lower end of an adjacent passage 17. An upper end of the adjacent passage 17 is aligned with one of the inner set of elongate recesses 23 of the upper manifold block 21, which in turn connects it to an upper end of a further passage 17. Thus, the carrier gas flows alternately upwardly and downwardly through each of the passages 17.

This allow heat transfer from the incoming carrier gas to the core block 2 and thence to the wicks 3, 4, 6 and the anaesthetic evaporating therefrom. Normally, such evaporation leads to a marked localised cooling, reducing the saturated vapour pressure of the anaesthetic in the carrier gas and reducing the rate of evaporation. This heat transfer arrangement thus raises the potential delivery rate of anaesthetic vapour from the wick assembly 1 and reduces its variability.

A last elongate recess 27 in the upper manifold block 21 connects a final one of the passages 17 to the central aperture 10 of the core block 2. Here, carrier gas absorbs, as it flows downwardly, anaesthetic vapour from the centre wick 6. The slot 11 in the centre wick 6 allows the gas to pass outwardly of the wick 6, above a point where the wick 6 enters the liquid anaesthetic held in the lower zone 20 of the reservoir chamber 18.

The carrier gas then passes upwardly through a passage port 28 extending through the base plate 13, and enters a channel 7 aligned therewith. An upper end of the channel 7 is linked via one of the outer set of elongate recesses 22 of the upper manifold block 21 to an upper end of an adjacent channel 7. The lower end of this adjacent channel 7 is similarly linked by one of the outer set of elongate recesses 15 of the base plate 13 to a lower end of a further channel 7, and so forth. The carrier gas flows alternately upwardly and downwardly through each of the channels 7.

This prolonged contact with wick material soaked in liquid anaesthetic ensures that the carrier gas becomes thoroughly saturated with anaesthetic vapour. The successive upward and downward flows ensure that there is no appreciable variation in vapour content due to it being heavier than the carrier gas, such as may occur with existing wick arrangements. In any case, there are no "short-cuts" through this assembly, such as may be possible in existing wick arrangements.

Thus, carrier gas exiting a final one of the channels 7, along arrow 25b, and being led away through an exit port 29 in the upper manifold block 21 aligned therewith, is reliably fully saturated with anaesthetic vapour.

A further problem, addressed by the wick assembly 1 shown, is "pumping". In this, the use of a mechanical ventilator to deliver an ultimate anaesthetic blend to a patient may cause pulsations in back-pressure in the carrier gas stream, as far back in the anaesthetic delivery system as the vaporiser. This can affect both carrier gas flow patterns and the saturated vapour pressure of the anaesthetic vapour in the carrier gas, leading to undesirable variations in the supply of anaesthetic vapour and carrier gas from the vaporiser.

Existing solutions include incorporating large gas volumes within a vaporiser, to buffer any pressure variations, or "anti-pumping coils" inserted into the carrier gas path outside the vaporiser. The first solution wastes valuable space within the vaporiser and the latter is an inconvenient extra piece of apparatus. However, in the present wick assembly 1, the long, relatively restricted carrier gas path extending through each of the passages 17 also acts as an antipumping coil, greatly reducing the effect of back pressure variations from downstream of the vaporiser, without using up valuable space within the vaporiser.

The vaporiser shown is a by-pass type vaporiser, in which an incoming carrier gas stream is divided into two, one sub-stream being passed through a wick arrangement to become saturated with anaesthetic vapour and the other sub-stream by-passing the wick arrangement. When the sub-streams are recombined, immediately before leaving the vaporiser, the carrier gas stream produced thus has a known partially-saturated anaesthetic vapour content.

However, as mentioned above, any wick arrangement, even the wick assembly 1 of the present invention, will cool to some extent when in use. The saturated vapour pressure of anaesthetic in the carrier gas will thus fall until the vaporiser reaches an equilibrium operating temperature. To achieve a constant net vapour content in the recombined carrier gas stream, more of the carrier gas must be routed through the wick assembly to pick up anaesthetic and less must by-pass it.

It is hence customary to incorporate a temperature compensating valve into the path of the carrier gas by-passing the wick. This valve incorporates a bimetallic strip which closes the valve as it cools, restricting flow through the valve and diverting more of the carrier gas through the wick arrangement. In existing vaporisers, this valve is mounted to an underside of the reservoir chamber 18, so that the bimetallic strip responds to the temperature of the liquid anaesthetic therein. However, the temperatures of the liquid anaesthetic in the reservoir and of the anaesthetic vapour in the wick arrangement can differ significantly.

Therefore, the vaporiser according to a preferred embodiment of the present invention is provided with a temperature compensating valve 30 mounted adjacent an upper part of the main body 19, and hence close to the wick assembly 1, its core block 2, and the carrier gas and anaesthetic vapour passing therethrough.

The temperature compensating valve 30 is mounted to a front block 31 of the vaporiser. It comprises a pair of closely-spaced circular plates 32, the opposing faces which are machined to a very close tolerance. Carrier gas enters a volume between the plates 32 adjacent their centres and flows radially outwardly. A bimetallic strip 33 is so arranged that, as it cools, it bears on one of the plates 32, and moves them together. The plates 32 are springloaded so that they move apart again if the bimetallic strip 33 warms and retracts.

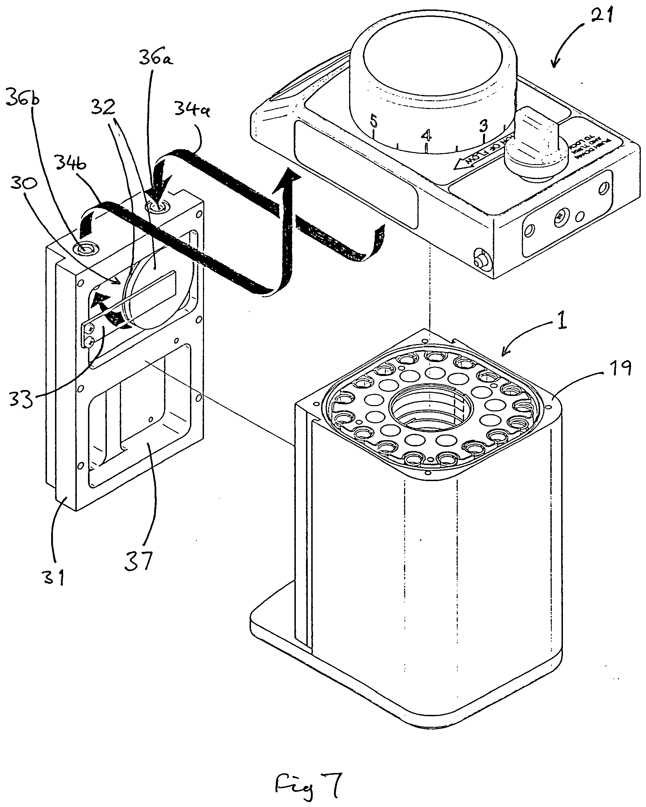

The carrier gas entering the vaporiser is split into two within the upper manifold block 21. The sub-stream by-passing the wick assembly 1 flows out of a first by-pass port 35a in the upper mounting body (see Figure 7) and enters the front block 31 through its corresponding first by-pass port 36a, as shown by arrow 34a. The sub-stream then passes through the restricted volume between the plates 32, and leaves the front block 31 through a second by-pass port 36b, re-entering the upper manifold block 21 through its corresponding second by-pass port 35b, as shown by arrow 34b. This sub-stream is then recombined, within the upper manifold block 21, with the sub-stream that has passed through the wick assembly 1.

This arrangement provides far more responsive compensation for any temperature variations within the wick assembly 1 than do conventional by-pass valve arrangements, and so delivers a significantly more consistent anaesthetic vapour concentration.

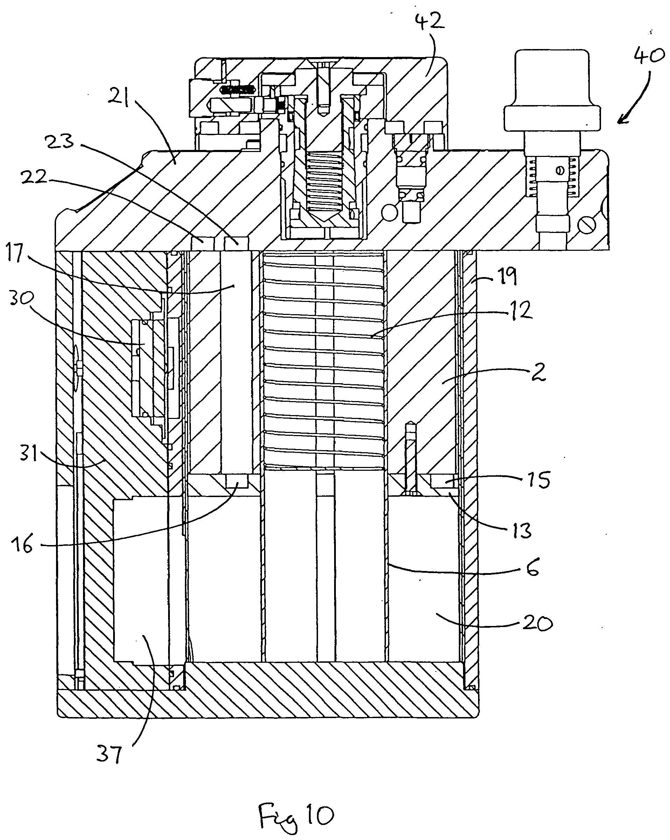

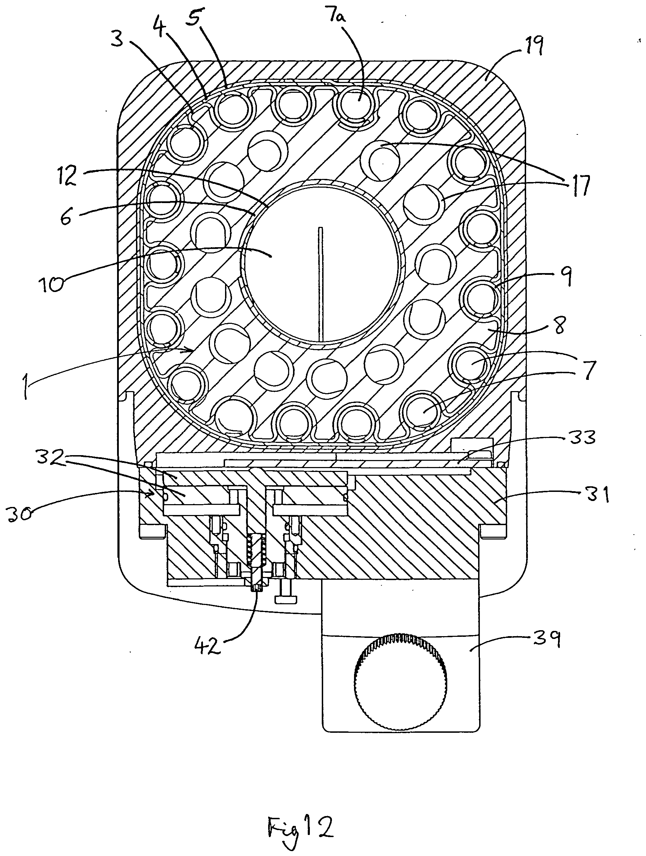

An additional benefit of this arrangement is that adjustment of the temperature compensating valve 30 is much easier than for a valve buried beneath the reservoir chamber 18 (see Figures 10 and 12 below for details).

As the valve 30 is not occupying space within the main body 19 beneath the reservoir chamber 18, this can be deeper for a given overall height of the vaporiser. Also, with the valve 30 occupying only an upper part of the front block 31, a lower part of the front block 31 can be hollowed out to form an extension 37 of the reservoir chamber 18, further increasing the capacity of the vaporiser without exceeding its maximum possible external dimensions. The embodiment shown has a capacity of 400cm³; equivalent conventional vaporisers can hold no more than 250cm³.

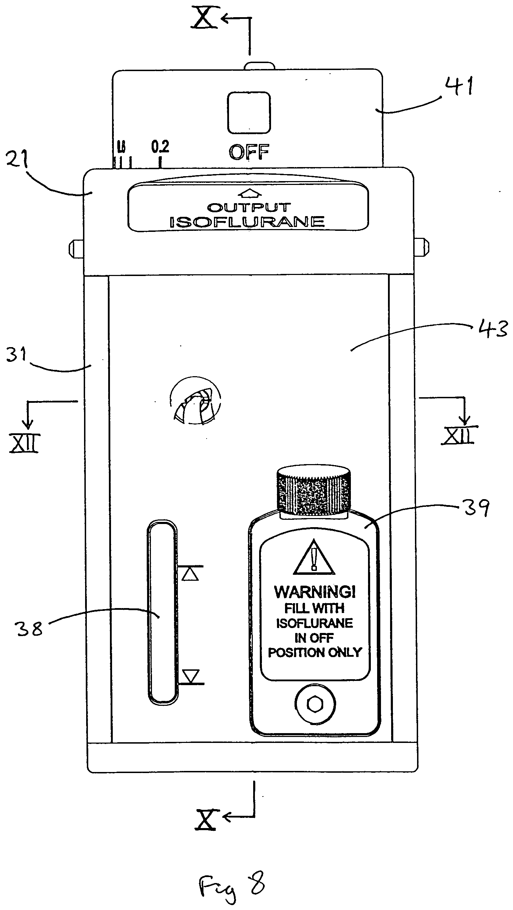

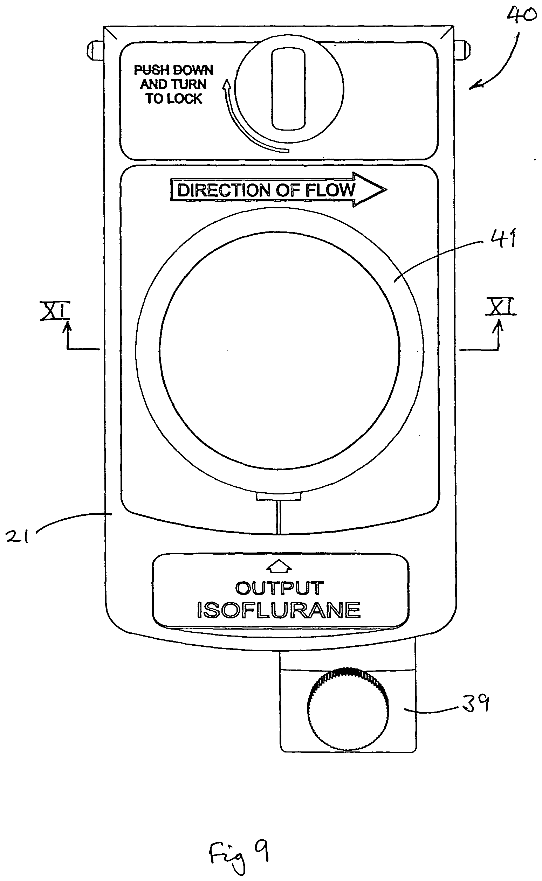

Figures 8 and 9 show the assembled vaporiser. A sight glass 38 is provided, allowing direct visualisation of the level of liquid anaesthetic in the reservoir chamber 18 and its extension 37. The sight glass 38 is provided with indicia showing a maximum fill level and a minimum fill level, below which the reservoir chamber 18 requires urgent replenishment.

A pour filler unit 39, of conventional form, is provided, mounted to the front block 31 and connected to the extension 37. This allows easy topping-up of the reservoir chamber 18 while the vaporiser is in use. Alternative filling systems can be used in place of the pour filler unit 39 shown.

The vaporiser shown has a conventional mounting arrangement 40 incorporated into the upper manifold block 21, so that it may be mounted to a desired existing mounting rack system (in this case, a Selectatec (Registered Trade Mark) system).

The net anaesthetic vapour content provided by the vaporiser is controlled by a conventional control valve arrangement within the upper manifold block 21, and is set by means of a control knob 41.

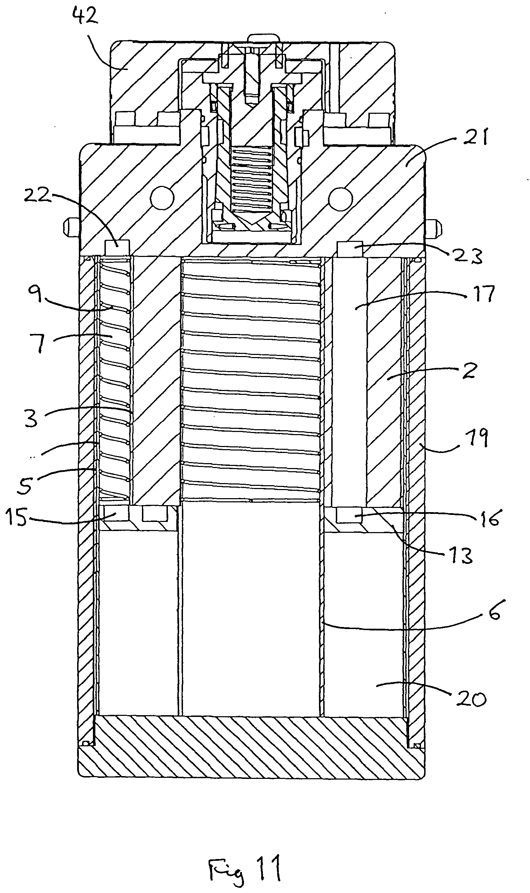

Figures 10 to 12 show the assembled vaporiser in cross-section. In particular, Figure 12 shows how close the bimetallic strip 33 of the temperature compensating valve 30 is to the wick assembly 1 and the channels 7 therethrough, ensuring that it responds reliably to the actual temperature of the carrier gas and anaesthetic vapour therein. Figure 12 also shows an adjusting body 32 for fine adjustment of the valve 30, and 42 for setting the maximum bypass resistance, easily accessible by removing a decorative fascia 43 from the front block 31.

The vaporiser shown provides a supply of anaesthetic vapour, borne in a stream of carrier gas, having a consistent, predetermined anaesthetic content. It undergoes lower temperature variations during operations than do existing vaporisers, and compensates more accurately and rapidly for any variations that do occur. It is easy to keep topped up during prolonged use, and in any case has a significantly greater anaesthetic capacity while having external dimensions compatible with existing mountings. It also has an in-built resistance to "pumping" effects due to mechanical ventilators connected thereto, and the anti-pumping design of the core also provides improved heat transfer.

## Claims

1. An anaesthetic vaporiser (1) comprising reservoir means (18) adapted to hold a supply of liquid anaesthetic agent, and **characterised by** a plurality of spaced, generally parallelly-extending channel means (7), each extending generally vertically, in use, to define a path for carrier gas being passed through the vaporiser (1), and each being provided with respective wick means (3) extending into said reservoir means (18) or operatively connected thereto so as to permit flow of liquid anaesthetic agent from the reservoir means (18) to each wick means (3).

2. A vaporiser (1) as claimed in claim 1, **characterised in that** the plurality of channel means (7) are so connected that the carrier gas flow is directed upwardly along some of said channel means (7) and downwardly along a remainder thereof, optionally alternately upwardly and downwardly along successive channel means (7).

3. A vaporiser (1) as claimed in either claim 1 or claim 2, **characterised in that** each said wick means (3) comprises a layer of wick material lining at least part of an inner surface of a respective channel means (7), optionally a majority thereof.

4. A vaporiser (1) as claimed in claim 3, **characterised in that** each channel means (7) is provided with frame means (9) adapted to hold said layer of wick material to said surface.

5. A vaporiser (1) as claimed in claim 4, **characterised in that** each channel means (7) has an elongate generally cylindrical shape and the frame means (9) comprise helical spring means (9) dimensioned to be insertable into a respective channel means (7) with the wick material held between it and the inner surface of the channel means (7).

6. A vaporiser (1) as claimed in any one of the preceding claims, **characterised in that** the wick means (3) are operatively connected with the reservoir means (18) by a sheet of second wick material (4) extending into said reservoir means (18), and optionally lining a part of the inner surface of each channel means (7), and contacting each wick means (3).

7. A vaporiser (1) as claimed in any one of the preceding claims, **characterised by** further comprising upper (21) and lower (13) end caps, each provided with passage means (22, 15) to connect a respective end of each channel means (7) to a corresponding end of another channel means (7), or to inlet (28) or outlet (29) means for the carrier gas.

8. An anaesthetic vaporiser (1) as claimed in any one of the preceding claims, **characterised in that** it further comprises a second carrier gas path avoiding contact between carrier gas and anaesthetic vapour, and temperature sensitive valve means (30) adapted to regulate the proportion of carrier gas flowing along each said path, the valve means (30) being so mounted as to sense and respond to the temperature of carrier gas and anaesthetic vapour adjacent an upper part of the reservoir means (18).

9. A vaporiser (1) as claimed in claim 8, **characterised in that** the valve means (30) comprises a variable restriction in the second carrier gas path, optionally a pair of plate means (32) between which the carrier gas is constrained to flow, and spaced by a separation controlled by movement of a bimetallic strip means (33).

10. An anaesthetic vaporiser (1) as claimed in any one of the preceding claims, **characterised in that** it further comprises a third carrier gas path having an exit (27) connected to an entry (28) to the first carrier gas path and extending so adjacent thereto that heat may flow from carrier gas in the third path to replace that lost by evaporation of anaesthetic into carrier gas in the first path.

11. A vaporiser (1) as claimed in any one of the preceding claims, **characterised in that** it further comprises buffer means to obviate the effects of variations in back-pressure, such as produced by a mechanical ventilator, operatively connected thereto, the buffer means comprising an elongate indirect carrier gas path within the vaporiser (1).

## Patentansprüche

1. Narkosemittelverdampfer (1), umfassend ein Behältermittel (18), das zum Halten einer Zufuhr von flüssigem Narkosemittel ausgelegt ist, und **gekennzeichnet ist durch** mehrere beabstandete, sich im Allgemeinen parallel erstreckende Kanalmittel (7), die sich im Allgemeinen jeweils vertikal erstrecken, um im Gebrauch einen Pfad für durch den Verdampfer (1) durchlaufendes Trägergas zu definieren, und die jeweils mit einem jeweiligen Dochtmittel (3) versehen sind, das sich in das Behältermittel (18) erstreckt oder damit wirkverbunden ist, um zu ermöglichen, dass flüssiges Narkosemittel von dem Behältermittel (18) zu jedem Dochtmittel (3) strömt.

2. Verdampfer (1) nach Anspruch 1, **dadurch gekennzeichnet, dass** die mehreren Kanalmittel (7) so verbunden sind, dass der Trägergasstrom entlang einiger der Kanalmittel (7) nach oben und entlang eines Rests davon nach unten, optional abwechselnd entlang aufeinanderfolgender Kanalmittel (7) nach oben und nach unten geleitet wird.

3. Verdampfer (1) nach Anspruch 1 oder 2, **dadurch gekennzeichnet, dass** jedes Dochtmittel (3) eine Schicht aus Dochtmaterial umfasst, das wenigstens einen Teil einer Innenoberfläche eines entsprechenden Kanalmittels (7), optional einen Großteil davon, auskleidet.

4. Verdampfer (1) nach Anspruch 3, **dadurch gekennzeichnet, dass** jedes Kanalmittel (7) mit einem Rahmenmittel (9) versehen ist, das ausgelegt ist, die Schicht aus Dochtmaterial auf der Oberfläche zu halten.

5. Verdampfer (1) nach Anspruch 4, **dadurch gekennzeichnet, dass** jedes Kanalmittel (7) eine längliche, im Allgemeinen zylindrische Form aufweist und die Rahmenmittel (9) ein Schraubenfedermittel (9) umfassen, das bemessen ist, in ein jeweiliges Kanalmittel (7) einführbar zu sein, wobei das Dochtmaterial zwischen diesem und der Innenoberfläche des Kanalmittels (7) gehalten wird.

6. Verdampfer (1) nach einem der vorhergehenden Ansprüche, **dadurch gekennzeichnet, dass** die Dochtmittel (3) durch eine Lage aus zweitem Dochtmaterial (4), das sich in das Behältermittel (18) hinein erstreckt und optional einen Teil der Innenoberfläche jedes Kanalmittels (7) auskleidet und jedes Dochtmittel (3) berührt, mit dem Behältermittel (18) wirkverbunden sind.

7. Verdampfer (1) nach einem der vorhergehenden Ansprüche, **dadurch gekennzeichnet, dass** er ferner obere (21) und untere (13) Endkappen umfasst, die jeweils mit einem Durchlassmittel (22, 15) versehen sind, um ein jeweiliges Ende jedes Kanalmittels (7) mit einem jeweiligen Ende eines anderen Kanalmittels (7), oder mit einem Einlass(28)- oder Auslass(29)-Mittel für das Trägergas zu verbinden.

8. Narkosemittelverdampfer (1) nach einem der vorhergehenden Ansprüche, **dadurch gekennzeichnet, dass** er ferner Folgendes umfasst: einen zweiten Trägergaspfad, der einen Kontakt zwischen Trägergas und Narkosedampf verhindert, und ein temperaturempfindliches Ventilmittel (30), das ausgelegt ist, das Verhältnis von entlang jedes Pfads strömendem Trägergas zu regeln, wobei das Ventilmittel (30) derart angebracht ist, dass es die Temperatur von Trägergas und Narkosedampf neben einem oberen Teil des Behältermittels (18) erfasst und auf diese reagiert.

9. Verdampfer (1) nach Anspruch 8, **dadurch gekennzeichnet, dass** das Ventilmittel (30) eine variable Begrenzung im zweiten Trägergaspfad umfasst, optional ein Paar von Plattenmitteln (32), zwischen denen das Trägergas beschränkt ist, zu strömen, und durch eine Trennung, die durch Bewegen eines Bimetallstreifenmittels (33) geregelt ist, beabstandet.

10. Narkosemittelverdampfer (1) nach einem der vorhergehenden Ansprüche, **dadurch gekennzeichnet, dass** er ferner einen dritten Trägergaspfad umfasst, der einen mit einem Eingang (28) verbundenen Ausgang (27) zum ersten Trägergaspfad aufweist und sich derart daran angrenzend erstreckt, dass Wärme vom Trägergas im dritten Pfad in ein Trägergas im ersten Pfad strömen kann, um jene durch Verdampfen eines Anästhetikums verlorene Wärme zu ersetzen.

11. Verdampfer (1) nach einem der vorhergehenden Ansprüche, **dadurch gekennzeichnet, dass** er ferner ein Puffermittel umfasst, um die Wirkungen von Abweichungen im Gegendruck, wie etwa durch einen mechanischen Ventilator erzeugt, der damit wirkverbunden ist, zu verhindern, wobei das Puffermittel einen länglichen indirekten Trägergaspfad im Verdampfer (1) umfasst.

## Revendications

1. Vaporisateur d'anesthésiant (1) qui comprend un moyen de réservoir (18) adapté pour contenir une alimentation d'agent anesthésiant liquide, et **caractérisé par** une pluralité de moyens de canal espacés s'étendant généralement parallèlement (7), chacun s'étendant généralement à la verticale, en utilisation, pour définir un trajet pour un gaz porteur passé à travers le vaporisateur (1), et chacun pourvu d'un moyen de mèche (3) respectif s'étendant dans ledit moyen de réservoir (18) ou raccordé opérationnellement à celui-ci de façon à permettre un écoulement d'agent anesthésiant liquide du moyen de réservoir (18) à chaque moyen de mèche (3).

2. Vaporisateur (1) selon la revendication 1, **caractérisé en ce que** la pluralité de moyens de canal (7) est raccordée de sorte que l'écoulement de gaz porteur soit dirigé vers le haut le long de certains desdits moyens de canal (7) et vers le bas le long d'un restant de ceux-ci, facultativement en alternance vers le haut et vers le bas le long de moyens de canal (7) successifs.

3. Vaporisateur (1) selon la revendication 1 ou la revendication 2, **caractérisé en ce que** chacun desdits moyens de mèche (3) comprend une couche de matériau de mèche garnissant au moins une partie d'une surface intérieure d'un moyen de canal (7) respectif, facultativement une majorité de celle-ci.

4. Vaporisateur (1) selon la revendication 3, **caractérisé en ce que** chaque moyen de canal (7) est pourvu d'un moyen de châssis (9) adapté pour maintenir ladite couche de matériau de mèche sur ladite surface.

5. Vaporisateur (1) selon la revendication 4, **caractérisé en ce que** chaque moyen de canal (7) a une forme cylindrique généralement allongée et le moyen de châssis (9) comprend un moyen de ressort hélicoïdal (9) dimensionné pour pouvoir être inséré dans un moyen de canal (7) respectif avec le matériau de mèche maintenu entre celui-ci et la surface intérieure du moyen de canal (7).

6. Vaporisateur (1) selon l'une quelconque des revendications précédentes, **caractérisé en ce que** le moyen de mèche (3) est raccordé opérationnellement au moyen de réservoir (18) par une feuille d'un second matériau de mèche (4) s'étendant dans ledit moyen de réservoir (18), et garnissant facultativement une partie de la surface intérieure de chaque moyen de canal (7), et venant en contact avec chaque moyen de mèche (3).

7. Vaporisateur (1) selon l'une quelconque des revendications précédentes, **caractérisé en ce qu'**il comprend en outre des embouts supérieur (21) et inférieur (13), pourvus chacun d'un moyen de passage (22, 15) pour raccorder une extrémité respective de chaque moyen de canal (7) à une extrémité correspondante d'un autre moyen de canal (7), ou à un moyen d'admission (28) ou de refoulement (29) pour le gaz porteur.

8. Vaporisateur d'anesthésiant (1) selon l'une quelconque des revendications précédentes, **caractérisé en ce qu'**il comprend en outre un deuxième trajet de gaz porteur évitant un contact entre un gaz porteur et une vapeur d'anesthésiant, et un moyen de valve sensible à la température (30) adapté pour réguler la proportion de gaz porteur s'écoulant le long de chaque dit trajet, le moyen de valve (30) étant monté de façon à détecter et répondre à la température de gaz porteur et de vapeur d'anesthésiant adjacent à une partie supérieure du moyen de réservoir (18).

9. Vaporisateur (1) selon la revendication 8, **caractérisé en ce que** le moyen de valve (30) comprend une restriction variable dans le deuxième trajet de gaz porteur, facultativement une paire de moyens de plaque (32) entre lesquels le gaz porteur est contraint de s'écouler, et espacés par une séparation commandée par un mouvement d'un moyen de bande bimétallique (33).

10. Vaporisateur d'anesthésiant (1) selon l'une quelconque des revendications précédentes, **caractérisé en ce qu'**il comprend en outre un troisième trajet de gaz porteur ayant une sortie (27) raccordée à une entrée (28) au premier trajet de gaz porteur et s'étendant adjacente à celle-ci de sorte que la chaleur puisse s'écouler depuis le gaz porteur dans le troisième trajet pour remplacer celle perdue par évaporation d'anesthésiant dans le gaz porteur dans le premier trajet.

11. Vaporisateur (1) selon l'une quelconque des revendications précédentes, **caractérisé en ce qu'**il comprend en outre un moyen de tampon pour éviter les effets de variation de contre-pression, tels que ceux produits par un ventilateur mécanique, raccordé opérationnellement à celui-ci, le moyen tampon comprenant un trajet de gaz porteur indirect allongé au sein du vaporisateur (1).
